# FASCICULE DE BREVET EUROPEEN

(11) **EP 3 559 056 B1**
(45) Date de publication et mention de la délivrance du brevet: **14.02.2024**
(21) Numéro de dépôt: 17832537.9
(22) Date de dépôt: 22.12.2017
(51) Int. Cl.: C08F 210/02, C08F 2/40, C08K 5/13

(54) **UTILISATION D'AU MOINS UN COMPOSÉ PHÉNOLIQUE POUR STABILISER DES RÉACTIONS DE COPOLYMÉRISATION D'ÉTHYLÈNE**
VERWENDUNG VON MINDESTENS EINER PHENOLISCHEN VERBINDUNG ZUR STABILISIERUNG VON ETHYLENCOPOLYMERISATIONSREAKTIONEN
USE OF AT LEAST ONE PHENOLIC COMPOUND TO STABILISE ETHYLENE COPOLYMERISATION REACTIONS

(30) Priorité: 23.12.2016 FR 1663341
(43) Date de publication de la demande: 30.10.2019
(73) Titulaire: SK Geo Centric Co., Ltd., Jongno-gu Seoul (KR)
(72) Inventeur: LAURICHESSE, Christian, 64140 Lons (FR); SEVERAC, Thibaut, 69100 Villeurbanne (FR); LELIEVRE, Angélique, 57690 Bambiderstroff (FR); MICHALOWICZ, Claire Isabelle, 2700 Evreux (FR); DEFOOR, Louis, 64140 Lons (FR); CABON, Yves, 57000 Metz (FR)
(74) Mandataire: Bandpay & Greuter
(86) Numéro de dépôt international: PCT/FR2017/053823
(87) Numéro de publication internationale: WO 2018/115790

(56) Documents cités:
- WO-A1-2007/135031
- WO-A1-2013/149698
- DE-A1- 2 946 954
- DE-A1-102009 023 651
- Y. C. HO ET AL: "Vitamin E based stabilizer components in HDPE polymer", JOURNAL OF VINYL AND ADDITIVE TECHNOLOGY, vol. 4, no. 2, 1 juin 1998 (1998-06-01), pages 139-150, XP055382454, US ISSN: 1083-5601, DOI: 10.1002/vnl.10031
- TOYODA J ET AL: "Push-pull modulation of ganglion cell responses of carp retina by amacrine cells", NEUROSCIENCE LETTERS, ELSEVIER, AMSTERDAM, NL, vol. 142, no. 1, 3 août 1992 (1992-08-03), pages 41-44, XP024349816, ISSN: 0304-3940, DOI: 10.1016/0304-3940(92)90615-E [extrait le 1992-08-03]

## Description

La présente invention concerne l'utilisation d'un ou plusieurs composés phénoliques, tels que définis ci-après, pour stabiliser les réactions de copolymérisation de l'éthylène sous haute pression.

L'invention concerne également un procédé de préparation d'un copolymère d'éthylène sous haute pression en présence d'un ou plusieurs composés phénoliques, tels que définis ci-après, et d'un ou plusieurs initiateurs.

Les polyéthylènes basse densité (encore appelé LDPE) et les copolymères de l'éthylène sont généralement préparés dans un réacteur autoclave ou tubulaire sous très haute pression, par introduction en continu d'éthylène, d'un ou plusieurs comonomères éventuels et d'un ou plusieurs initiateurs, tels que les peroxydes organiques, généralement dilués dans un solvant organique. La pression à l'intérieur du réacteur est généralement comprise entre 500 et 5000 bars tandis que la température, lors de l'initiation de la réaction, varie le plus souvent de 80 à 250°C. La température maximale de réaction est typiquement comprise entre 120 et 350°C.

Le taux de conversion en polymère couramment obtenu avec ce type de procédé est de l'ordre de 15 à 25%. De la même façon, la productivité d'un procédé, exprimée en grammes de polyéthylène ou copolymère d'éthylène produit par gramme d'initiateur peroxyde utilisé, peut osciller de 1000 à 3000 g/g, et est souvent inférieure à 2500 g/g.

La recherche de gain de production, et par conséquent de rationalisation des coûts, est une préoccupation constante des producteurs de polyéthylène et de copolymère de l'éthylène. En particulier, il est important de rechercher à mettre en oeuvre un procédé permettant la fabrication de polyéthylène ou de copolymères de l'éthylène qui présente une productivité élevée tout en conservant une bonne fiabilité.

Cette amélioration dans le gain de productivité est d'autant plus important que les polyéthylènes et les copolymères d'éthylène sont notamment intéressants commercialement car ils peuvent être utilisés dans des domaines d'application variés en raison de leur compatibilité avec de nombreux autres polymères ou résines.

A titre d'exemples, les copolymères d'éthylène peuvent être utilisés dans la fabrication de câbles, de compositions adhésives thermofusibles, de films d'emballage multicouches ou de mélanges maîtres. Ils peuvent être également utilisés comme modifiant choc dans la préparation de polymères tels que les polyamides et polyesters pour les secteurs de l'électronique et de l'automobile.

Cependant, les producteurs de polyéthylène et de copolymère de l'éthylène rencontrent fréquemment des phénomènes d'emballement de réaction et de décompositions thermiques de l'éthylène car de telles réactions sont conduites à haute température et haute pression.

En particulier, dans ces conditions opératoires, l'éthylène se décompose de façon explosive, au cours de la réaction de polymérisation en présence d'excès d'initiateurs (peroxydes organiques, oxygène) en conduisant à la formation de carbone, de méthane et d'hydrogène.

Ces réactions parasitaires de dégradation de l'éthylène dues à un excès d'initiateurs, dans les conditions opératoires mentionnées ci-avant, engendrent au mieux une production de granulés colorés (décomposition douce), au pire une montée en pression et température rapide (décomposition violente) dans le réacteur déclenchant un arrêt de la production et des opérations de maintenance coûteuses.

Ainsi l'un des objectifs de la présente invention est de réduire la fréquence de décomposition de l'éthylène au cours des procédés de copolymérisation de l'éthylène sous haute pression de manière à améliorer leur productivité et réduire les coûts de maintenance associés à ces décompositions.

En d'autres termes, il existe un réel besoin de mettre en oeuvre un procédé de copolymérisation de l'éthylène au cours duquel les réactions de copolymérisation de l'éthylène sous haute pression sont stabilisées en minimisant le risque de dégradation de l'éthylène.

Le document WO 2013/149698 décrit l'utilisation de composés phénoliques encombrés pour réduire l'encrassement des réacteurs de polymérisation de polyéthylène.

Ce document divulgue notamment qu'un mélange, constitué de l'éthylène et d'un ou plusieurs comonomères éventuels, est chauffé de manière à atteindre une température suffisante pour permettre aux peroxydes organiques, une fois introduits dans le mélange, de se décomposer en radicaux libres afin d'amorcer plus efficacement la polymérisation. Le mélange de monomères est ainsi préchauffé, avant l'introduction des peroxydes organiques, en début de réacteur dans des équipements prévus à cet effet.

Toutefois, au cours de cette étape de préchauffage, ce mélange réactionnel a une tendance à se polymériser aisément au niveau des parois du préchauffeur.

Cette polymérisation non désirée a pour conséquence de limiter le transfert de chaleur vers les peroxydes organiques ce qui les empêche de former des radicaux libres et de permettre le démarrage de la polymérisation dans des conditions opératoires optimales. Cette réaction parasitaire provoque un encrassement des parois du dispositif servant au préchauffage.

Les composés phénoliques encombrés sont ainsi ajoutés dans le mélange de monomères, avant l'introduction des peroxydes organiques, afin de réduire, voire d'éliminer, la polymérisation parasitaire ayant lieu au cours de l'étape de préchauffage et, par conséquent, de réduire l'encrassement en début de réacteur. En d'autres termes, les composés phénoliques sont employés pour réduire la polymérisation parasitaire, ayant lieu dans le mélange de monomères, avant que la polymérisation radicalaire, amorcée par les peroxydes organiques, ne démarre dans la zone réactionnelle.

Ce document ne décrit donc pas l'utilisation de composés phénoliques pour diminuer la fréquence de décompositions de l'éthylène au cours de 1a polymérisation sous haute pression, c'est-à-dire en présence de peroxydes organiques jouant le rôle d'initiateurs de polymérisation.

La présente invention a donc notamment pour objet l'utilisation d'au moins un composé phénolique de formule (I) suivante :

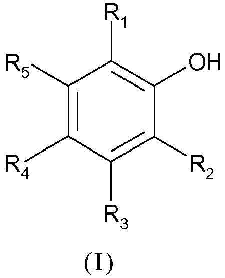

Formule (I) dans laquelle :
- R¹ représente :
   - un atome d'hydrogène,
   - un radical alkyle linéaire en C₁-C₈,
   - un radical hydroxyalkyle linéaire en C₁-C₈,
   - un groupement hydroxy,
- R², R³ et R⁵, identiques ou différents, représentent un :
   - atome d'hydrogène,
   - un radical alkyle en C₁-C₈, linéaire ou ramifié, éventuellement substitué par un ou plusieurs radicaux hydroxy,
   - un radical alcoxy en C₁-C₈, linéaire ou ramifié, éventuellement substitué par un ou plusieurs radicaux hydroxy,
   - un groupement hydroxy <Insertion page 4a>
- étant entendu que R⁴ et R⁵ peuvent former ensemble un cycle à cinq ou six chaînons comportant un atome d'oxygène ; ledit cycle étant substitué par un radical alkyle en C₁-C₁₇, linéaire ou ramifié,
pour stabiliser les réactions de copolymérisation radicalaire de l'éthylène sous haute pression en présence d'un ou plusieurs initiateurs le ou les initiateurs étant de préférence choisis parmi les peroxydes organiques, l'oxygène l'azobisisobutyronitrile (AIBN) et leurs mélanges, en limitant la décomposition de l'éthylène lors de réactions de copolymérisation radicalaire de l'éthylène sous haute pression.

Le ou les composés phénoliques de formule (I) ont pour avantage de diminuer de manière significative la fréquence de
- R⁴ représente :
   - un radical alkyle en C₁-C₈, linéaire ou ramifié, éventuellement substitué par un ou plusieurs radicaux hydroxy,
   - un radical alcoxy en C₁-C₈, linéaire ou ramifié, éventuellement substitué par un ou plusieurs radicaux hydroxy,
   - un groupement hydroxy,
décomposition de l'éthylène, dans un mélange réactionnel comportant d'autres monomères, au cours de la copolymérisation radicalaire de l'éthylène sous haute pression en présence d'initiateurs.

En d'autres termes, le ou les composés phénoliques selon l'invention permettent de diminuer la sensibilité de l'éthylène à la concentration en initiateurs (tels que les peroxydes organiques, l'oxygène) limitant ainsi leur dégradation en carbone, méthane et hydrogène.

Plus généralement, le ou les composés phénoliques conformes à l'invention permettent d'améliorer la productivité des procédés de préparation de copolymères d'éthylène tout en minimisant les coûts de maintenance.

En particulier, le ou les composés phénoliques, conformes à l'invention, présentent l'avantage de réduire de manière significative la fréquence de décomposition de l'éthylène par rapport à l'utilisation de l'hydroxytoluène butylé dans les mêmes conditions.

L'invention concerne également un procédé de préparation de copolymères d'éthylène comprenant une étape de copolymérisation radicalaire de l'éthylène sous haute pression en présence d'un ou plusieurs initiateurs et d'un ou plusieurs composés phénoliques tels que décrits ci-avant- ,le procédé étant caractérisé en ce qu'il ne comprend pas d'étape de préchauffage des monomères avant l'introduction dudit un ou plusieurs initiateurs.

Le procédé de préparation conforme à l'invention présente une productivité élevée et une meilleure fiabilité.

En effet, les réactions parasitaires de dégradation de l'éthylène, entravant la productivité d'un procédé de préparation classique, sont diminuées.

Est également décrite une composition polymère obtenue par copolymérisation radicalaire de l'éthylène sous haute pression en présence d'un ou plusieurs initiateurs et d'un ou plusieurs composés phénoliques de formule (I).

Par « haute pression », on entend au sens de la présente invention, une pression supérieure à 50 MPa. De préférence, la pression varie de 500 bar (50 MPa) à 3000 bar (300 MPa), préférentiellement de 1200 bar (120 MPa) à 3000 bar (300 MPa), mieux de 1200 bar (120 MPa) à 2600 bar (260 MPa).

D'autres caractéristiques et avantages de l'invention apparaîtront plus clairement à la lecture de la description et des exemples qui suivent.

Dans ce qui va suivre, et à moins d'une autre indication, les bornes d'un domaine de valeurs sont comprises dans ce document.

L'expression « au moins un » est équivalente à l'expression « un ou plusieurs ».

### Composé phénolique

Par composé phénolique, on entend au sens de la présente invention, un composé comprenant au moins un phénol dans sa structure.

Le ou les composés phénoliques, utilisés pour stabiliser les réactions de copolymérisation radicalaire de l'éthylène sous haute pression, répondent à la formule (I) telle que décrite précédemment.

Conformément à la formule (I), R¹ ne représente pas un radical alkyle en C₁-C₈ ramifié.

Conformément à la formule (I), R⁴ et R⁵ peuvent former ensemble un cycle à cinq ou six chainons comportant un atome d'oxygène ; ledit cycle étant substitué par un radical alkyle en C₁-C₁₇, linéaire ou ramifié.

Ainsi le cycle phénol peut être condensé sur un cycle à cinq ou six chainons comportant un atome d'oxygène ; ledit cycle étant substitué par un radical alkyle en C₁-C₁₇, linéaire ou ramifié, de préférence ramifié.

De préférence, le ou les composés phénoliques répondent à la formule (I) suivante :

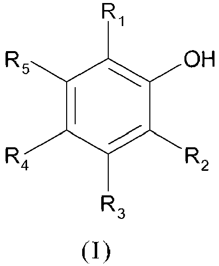

Formule (I) dans laquelle :
- R'représente :
   - un atome d'hydrogène,
   - un radical alkyle linéaire en C₁-C₄,
- R² et R³, identiques ou différents, représentent :
   - un atome d'hydrogène,
   - un radical alkyle, linéaire ou ramifié, en C₁-C₅,
   - un groupement hydroxy, <Insertion page 7a>
- R⁵ représente :
   - un atome d'hydrogène,
   - un radical alkyle, linéaire ou ramifié, en C₁-C₅,
   - un radical alcoxy, linéaire ou ramifié, en C₁-C₅,
   - un groupement hydroxy,
- étant entendu que R⁴ et R⁵ peuvent former ensemble un cycle à six chainons comportant un atome d'oxygène ; ledit cycle étant substitué par un radical alkyle ramifié en C₁-C₁₇.

De préférence, dans la formule (I) :
- R¹, R² et R³, identiques ou différents, représentent :
   - un atome d'hydrogène,
   - un radical alkyle linéaire en C₁-C₄, et
- R⁴ et R⁵ forment ensemble un cycle à six chainons comportant un atome d'oxygène ; ledit cycle étant substitué par un radical alkyle ramifié en C₁-C₁₇.

Plus préférentiellement, dans la formule (I) :
- R¹, R² et R³, identiques ou différents, représentent :
   - un atome d'hydrogène,
   - un radical méthyle, et
- R⁴ représente :
   - un radical alkyle, linéaire ou ramifié, en C₁-C₅,
   - un groupement hydroxy,
- R⁴ et R⁵ forment ensemble un cycle à six chaînons comportant un atome d'oxygène ; ledit cycle étant substitué par un radical alkyle ramifié en C₁-C₁₇.

Conformément à ces modes de réalisation, R¹, R² et R³ peuvent représenter préférentiellement un radical alkyle linéaire en C₁-C₄, en particulier un radical méthyle.

Conformément à ces modes de réalisation, R¹ et R³ peuvent représenter préférentiellement un radical alkyle linéaire en C₁-C₄, en particulier un radical méthyle, et R² représente un atome d'hydrogène.

Conformément à ces modes de réalisation, R² et R³ peuvent représenter préférentiellement un radical alkyle linéaire en C₁-C₄, en particulier un radical méthyle, et R¹ représente un atome d'hydrogène.

Conformément à ces modes de réalisation, R¹ et R² peuvent représenter préférentiellement un atome d'hydrogène et R³ représente un radical alkyle linéaire en C₁-C₄, en particulier un radical méthyle.

En d'autres termes, le ou les composés phénoliques est ou sont préférentiellement choisis parmi la formule (I') suivante :

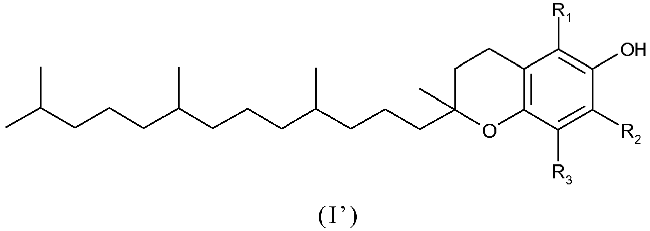

Formule (I') dans laquelle R¹, R² et R³ correspondent aux significations précédemment décrites, notamment celles mentionnées ci-avant dans les modes de réalisation préférentiels.

De préférence, en variante, dans la formule (I) :
- R¹, R², R³ et R⁵ représentent un atome d'hydrogène, et
- R⁴ représente un radical alcoxy linéaire en C₁-C₅, de préférence un radical méthoxy.

Encore de préférence, en variante, dans la formule (I) :
- R¹ et R³ représentent un atome d'hydrogène,
- R² et R⁵ représentent un radical alkyle ramifié en C₁-C₈, de préférence en C₁-C₅ ramifié, notamment en C₄,
- R⁴ représente un groupement hydroxy.

Préférentiellement, le composé phénolique de formule (I) est choisi parmi le 2,5-di-tert-butyl hydroquinone, le 2,5-di(tert-amyl)hydroquinone, la vitamine E et le monométhyl éther d'hydroquinone (EMHQ) et leurs mélanges.

Plus préférentiellement, le ou les composés phénoliques selon l'invention est ou sont choisis parmi la vitamine E et le monométhyl éther d'hydroquinone (EMHQ).

Encore plus préférentiellement, le composé phénolique selon l'invention est la vitamine E.

En d'autres termes, le composé phénolique selon l'invention correspond à la formule (I').

### Utilisation

Le ou les composés phénoliques de formule (I) sont utilisés pour stabiliser les réactions de copolymérisation radicalaire de l'éthylène sous haute pression en présence d'un ou plusieurs initiateurs.

Autrement dit, le ou les composés phénoliques de formule (I) sont utilisés pour stabiliser les réactions de copolymérisation radicalaire de l'éthylène sous haute pression initiées par un ou plusieurs initiateurs.

Encore en d'autres termes, le ou les composés phénoliques de formule (I) est ou sont utilisés dans un mélange réactionnel comprenant au moins l'éthylène et un ou plusieurs initiateurs pour stabiliser les réactions de copolymérisation radicalaire de l'éthylène sous haute pression.

Par « stabiliser les réactions de copolymérisation radicalaire de l'éthylène », on entend au sens de la présente invention la réduction des réactions de décomposition (ou dégradation) de l'éthylène en carbone, hydrogène et méthane ayant lieu lors de la réaction de copolymérisation radicalaire de l'éthylène et provoquées par un excès d'initiateurs (tels que les peroxydes organiques et/ou l'oxygène).

Autrement dit, le ou les composés phénoliques de formule (I) est ou sont utilisés pour limiter la décomposition de l'éthylène lors de réactions de copolymérisation radicalaire de l'éthylène sous haute pression.

Plus précisément, le ou les composés phénoliques est ou sont utilisés pour limiter le nombre et la vitesse de réaction de décomposition de l'éthylène en carbone, hydrogène et méthane lors de réactions de copolymérisation radicalaire de l'éthylène sous haute pression qui sont initiées par un ou plusieurs initiateurs.

Le ou les initiateurs permettent de former des radicaux libres afin d'initier la copolymérisation radicalaire de l'éthylène.

En d'autres termes, le ou les initiateurs permettent d'amorcer la copolymérisation radicalaire de l'éthylène.

Le ou les initiateurs peuvent être choisis parmi les peroxydes organiques, l'oxygène, l'azobisisobutyronitrile (AIBN) et leurs mélanges.

De préférence, le ou les initiateurs est ou sont choisis parmi les peroxydes organiques, l'oxygène et leurs mélanges.

Encore plus préférentiellement, le ou les initiateurs est ou sont choisis parmi les peroxydes organiques.

De préférence, les peroxydes organiques sont choisis parmi les peroxy esters, les peroxydes de dialkyle, les hydroperoxydes ou les peroxycétals.

De tels peroxydes sont notamment commercialisés par la société Arkema sous la dénomination commerciale Luperox^{®}.

A titre d'exemples de peroxy esters, on peut citer le t-butyl peroxy-2-éthylhexanoate (Luperox 26), le t-butyl peroxyacétate (Luperox 7), le t-amyl peroxyacétate (Luperox 555), le t-butyl perbenzoate (Luperox P), le t-amyl perbenzoate (Luperox TAP) et le OO-t-butyl 1-(2-éthylhexyl)monoperoxycarbonate (Luperox TBEC).

A titre d'exemples de peroxydes de dialkyle, on peut citer le 2,5-diméthyl-2,5-di-(t-butylperoxy)hexane (Luperox 101), le dicumyl peroxyde (Luperox DC), l'alpha-alpha'-bis (t-butylperoxy) diisopropylbenzene (Luperox F40), le di-t-butyl-peroxyde (Luperox DI), le di-t-amyl- peroxyde (Luperox DTA) et le 2,5-diméthyl-2,5-di-(t-butylperoxy)hexyne-3 (Luperox 130).

A titre d'hydroperoxyde, on peut citer le tert-butyl-hydroperoxyde (Luperox TBH 70).

A titre d'exemple de peroxycétals, on peut citer le l,l-di-(t-butylperoxy)-3,3,5- triméthylcyclohexane (Luperox 231 ), l'éthyl-3,3-di-(t-butylperoxybutyrate) (Luperox 233) ou l'éthyl-3,3-di-(t-amylperoxybutyrate) (Luperox 533).

Préférentiellement, les peroxydes organiques sont choisis parmi les peroxydes de dialkyle, en particulier le 2,5-diméthyl-2,5-di-(t-butylperoxy)hexane vendu sous la dénomination commerciale Luperox 101.

Le ou les peroxydes organiques est ou sont généralement dilués dans un solvant ou un mélange de solvants. Le ou les solvants peuvent être choisis parmi les alcanes en C₁-C₂₀, en particulier en C₃-C₁₀, et plus particulièrement en C₅-C₈, et préférentiellement l'heptane.

Le ou les composés phénoliques de formule (I) sont notamment utilisés pour stabiliser les réactions de copolymérisation radicalaire de l'éthylène avec d'autres comonomères.

De préférence, lesdits comonomères sont choisis parmi les esters d'acides carboxyliques insaturés (ou leurs sels), les anhydrides d'acides carboxyliques, les esters vinyliques, tels que l'acétate de vinyle ou l'acétate de pivalate, les alpha-oléfines tels que le propène, le 1-butène, le 1-hexène, le 1-octène et le 4-méthyl-1-pentène, les acides carboxyliques insaturés tels que l'acide (méth)acrylique, l'acide maléique et l'acide fumarique, les dérivés d'acides (méth)acryliques tels que le (méth)acrylonitrile et l'amide de (méth)acrylique, les éthers de vinyle tels que le vinyle méthyle éther et le vinyle phényle éther et les composé aromatiques vinyliques tels que le styrène et l'alphaméthyle styrène, le monoxyde de carbone ou leurs mélanges.

Plus préférentiellement, le ou les comonomères sont choisis parmi les esters d'acides carboxyliques insaturés (ou leurs sels), les anhydrides d'acides carboxyliques et leurs mélanges.

Les esters d'acides carboxyliques insaturés sont de préférence choisis parmi les (méth)acrylates d'alkyles, en particulier les (méth)acrylates d'alkyles en C₁-C₂₄, et les (méth)acrylates comportant un groupement époxy.

De préférence, les (méth)acrylates d'alkyles sont choisis parmi l'acrylate de méthyle, l'acrylate d'éthyle, l'acrylate de n-butyle, l'acrylate d'isobutyle, l'acrylate d'éthyl-2-hexyle, l'acrylate de cyclohexyle, l'acrylate de n-octyle, le méthacrylate de méthyle, le méthacrylate d'éthyle et le méthacrylate de butyle.

De préférence, les (méth)acrylates comportant un groupement époxy sont choisis parmi le méthacrylate de glycidyle et l'acrylate de glycidyle.

Préférentiellement, le ou les composés phénoliques de formule (I) sont utilisés pour stabiliser les réactions de copolymérisation radicalaire de l'éthylène avec des comonomères choisis parmi un mélange d'esters d'acides carboxyliques insaturés et les anhydrides d'acides carboxyliques en présence d'un ou plusieurs initiateurs.

Encore plus préférentiellement, le ou les composés phénoliques de formule (I) sont utilisés pour stabiliser les réactions de copolymérisation de l'éthylène avec un mélange de comonomères contenant l'acrylate de méthyle et méthacrylate de glycidyle.

De manière encore plus préférentielle, le ou les composés phénoliques de formule (I) sont utilisés pour stabiliser les réactions de copolymérisation radicalaire entre l'éthylène et un mélange de comonomères contenant l'acrylate de méthyle et méthacrylate de glycidyle en présence d'un ou plusieurs initiateurs choisis parmi les peroxydes organiques, l'oxygène ou leurs mélanges.

### Procédé

Comme indiqué ci-avant, l'invention concerne aussi un procédé de préparation de copolymères d'éthylène comprenant une étape de copolymérisation radicalaire de l'éthylène sous haute pression en présence d'un ou plusieurs initiateurs, tels que décrits ci-avant, et d'un ou plusieurs composés phénoliques tels que décrits ci-avant.

Le ou les initiateurs est ou sont préférentiellement choisis parmi les peroxydes organiques, l'oxygène et leurs mélanges.

De préférence, le ou les composés phénoliques selon l'invention est ou sont choisis parmi la vitamine E et le monométhyl éther d'hydroquinone (EMHQ).

Encore plus préférentiellement, le composé phénolique selon l'invention est la vitamine E. Autrement dit, le composé phénolique répond à la formule (I') précédente.

Le ou les composés phénoliques est ou sont, de préférence présents dans une quantité massique comprise entre 100 et 50000 ppm par rapport à la quantité massique de comonomères.

De préférence, le ou les composés phénoliques est ou sont solubilisés dans un solvant organique, de préférence un solvant organique de type hydrocarbure, alcool ou cétone, encore préférentiellement solvant organique de type hydrocarbure, en particulier l'isododécane, avant d'être introduit dans le réacteur, de préférence dans une quantité massique comprise entre 5 et 80% en poids, par rapport à la quantité massique de solvant.

Alternativement, le ou les composés phénoliques est ou sont solubilisés dans le comonomère avant d'être introduits dans le réacteur, de préférence dans une quantité massique comprise entre 100 et 50000 ppm par rapport à la quantité massique de comonomères.

Le ou les initiateurs est ou sont, de préférence, présents dans une quantité massique comprise entre 20 et 1000 ppm par rapport à la quantité massique d'éthylène.

La copolymérisation de l'éthylène se fait à une température d'initiation variant de 100 à 200°C, de préférence de 120 à 160°C.

La copolymérisation se fait à une pression variant de 500 bar (50 MPa) à 3000 bar (300 MPa), de préférence de 1200 bar (120 MPa) à 3000 bar (300 MPa), mieux de 1200 bar (120 MPa) à 2600 bar (260 MPa).

La copolymérisation à haute pression est généralement effectuée en réacteur autoclave ou tubulaire. La température de réaction est généralement comprise entre 150°C et 320°C.

Lorsqu'un réacteur tubulaire est utilisé, l'introduction du mélange de l'éthylène et du ou des comonomères est effectuée de préférence en tête du réacteur tubulaire. L'initiateur ou le mélange d'initiateurs est injecté à l'aide d'une pompe haute pression en tête du réacteur, après l'endroit d'introduction du mélange de l'éthylène et du ou des comonomères.

Le mélange de l'éthylène et du ou des comonomères éventuels peut être injecté en au moins un autre point du réacteur, cette injection est elle-même suivie d'une nouvelle injection d'initiateur ou d'un mélange d'initiateurs, on parle alors de technique d'injection multipoint. Lorsque la technique d'injection multipoint est utilisée, le mélange est préférentiellement injecté de manière telle que le rapport pondéral du mélange injecté en entrée de réacteur sur la totalité du mélange injecté est compris entre 10 et 90%.

D'autres procédés de polymérisation ou copolymérisation haute pression tubulaire utilisables sont par exemple ceux décrits dans US2006/0149004 A1 ou dans US2007/0032614 A1.

On peut également utiliser un réacteur autoclave pour réaliser la polymérisation haute pression radicalaire. Un réacteur autoclave consiste généralement en un réacteur cylindrique dans lesquels est placé un agitateur. Le réacteur peut être séparé en plusieurs zones reliées entre elles en série.

De préférence, le procédé conforme à l'invention est mis en oeuvre dans un réacteur autoclave.

Avantageusement, le temps de séjour dans le réacteur est compris entre 30 et 120 secondes.

Préférentiellement le rapport longueur/diamètre du réacteur est compris entre 3 et 25. L'éthylène et le ou les comonomères sont injectés dans la ou les zones réactionnelles à une température comprise entre 50 et 120°C.

De préférence, l'éthylène et le ou les comonomères sont injectés dans le ou les zones réactionnelles à une température strictement inférieure à 100°C, de préférence à une température strictement inférieure à 80°C.

De préférence, le procédé de préparation de copolymères d'éthylène selon l'invention ne comprend pas d'étape de préchauffage des monomères avant leur introduction dans le ou les zones réactionnelles.

De préférence, l'injection dudit un ou plusieurs initiateurs dans le mélange réactionnel débute à une température strictement inférieure à 100°C, de préférence à une température strictement inférieure à 80°C. Le procédé de préparation de copolymères d'éthylène selon l'invention ne comprend pas d'étape de préchauffage des monomères avant l'introduction dudit un ou plusieurs initiateurs.

Alternativement, un initiateur est également injecté dans cette première zone réactionnelle lorsque la zone réactionnelle atteint une température comprise entre 150 et 200°C.

Au cours de la réaction, la température peut être comprise entre 150 et 320°C, car la réaction est exothermique. Si le réacteur est un réacteur multizones, le flux d'éthylène et de comonomères éventuels n'ayant pas réagi ainsi que le polymère formé passent alors dans les zones réactionnelles suivantes.

Dans chaque zone réactionnelle, de l'éthylène, des comonomères et des initiateurs peuvent être injectés, à une température d'initiation comprise entre 150 et 200°C. La température des zones après l'initiation est comprise entre 150 et 320°C.

La pression du réacteur varie de 500 bar (50 MPa) à 3000 bar (300 MPa), préférentiellement de 1200 bar (120 MPa) à 3000 bar (300 MPa), mieux de 1200 bar (120 MPa) à 2600 bar (260 MPa).

Produit issu de la composition _polymérisable.

Est également décrit le produit issu de la copolymérisation radicalaire de l'éthylène sous haute pression en présence d'un ou plusieurs initiateurs, tels que décrits ci-avant, et d'un ou plusieurs composés phénoliques tels que décrits ci-avant.

Ainsi le produit est une composition polymère (ou un produit polymère) qui résulte de la copolymérisation radicalaire sous haute pression entre l'éthylène et d'autres comonomères en présence d'un ou plusieurs initiateurs, tels que décrits ci-avant, et d'un ou plusieurs composés phénoliques tels que décrits ci-avant.

Le produit polymère ou la composition polymère ainsi obtenu peut être utilisé dans tout type d'application, notamment dans le domaine de l'emballage, en particulier l'emballage alimentaire.

De préférence, le ou les initiateurs est ou sont préférentiellement choisis parmi les peroxydes organiques, l'oxygène et leurs mélanges

La composition polymère comprend le copolymère d'éthylène et le ou les composés phénoliques tels que décrits ci-avant.

De préférence, le ou les composés phénoliques est ou sont choisis parmi la vitamine E et le monométhyl éther d'hydroquinone (EMHQ).

Encore plus préférentiellement, la composition polymère comprend la vitamine E. Autrement dit, le composé phénolique répond à la formule (I') précédente.

Une fois le copolymère obtenu, un ou plusieurs additifs peu(ven)t être ajoutés à la composition polymère.

L'additif est de préférence choisi parmi les antioxydants ; les agents de protection UV ; les agents de mise en oeuvre, ayant pour fonction d'améliorer l'aspect final lors de sa mise en oeuvre, telles que les amides grasses, l'acide stéarique et ses sels, l'éthylène bisstéaramide ou les polymères fluorés ; les agents anti- buée ; les agents anti-bloquants tels que la silice ou le talc ; les charges telles que le carbonate de calcium et les nanocharges comme par exemple les argiles ; les agents de couplage tels que les silanes ; les agents réticulants comme les peroxydes différents de ceux employés en tant qu'amorceurs de copolymérisation radicalaire ; les agents antistatiques ; les agents nucléants ; les pigments ; les colorants ; les plastifiants ; les fluidifiants et les additifs retardateurs de flamme tels que les hydroxydes d'aluminium ou de magnésium.

Ces additifs sont généralement utilisés dans des teneurs comprises entre 10 ppm et 10 000 ppm en poids par rapport au poids de polyéthylène ou de copolymère d'éthylène final.

Les plastifiants, les fluidifiants et les additifs retardateurs de flamme peuvent atteindre des quantités bien supérieures à 10 000 ppm.

Les exemples suivants servent à illustrer l'invention sans toutefois présenter un caractère limitatif.

### EXEMPLES

Les exemples suivants ont été effectués sur un micro-pilote continu et agité de type autoclave d'un volume de 110 ml.

Cet équipement fonctionne en régime continu à des pressions comprises entre 500 et 2200 bars. La température de paroi du réacteur est fixée à 200°C à l'aide de crayons chauffants placés dans les parois du réacteur. L'agitation est de 1540 tr/min (tours par minute).

La température du milieu réactionnel dans le réacteur est mesurée à l'aide de quatre thermocouples.

Le mélange réactionnel est composé d'un mélange éthylène et d'acrylates qui entre en continu dans le réacteur avec un temps de résidence pouvant varier de 30 secondes à 120 secondes. L'agent stabilisant (composé phénolique) est introduit en mélange avec les acrylates.

L'initiateur de polymérisation est introduit en continu dans le réacteur avec des quantités permettant d'atteindre une température de réaction voisine de 210°C. En sortie du réacteur, le mélange polymère/monomères est détendu directement à trois bars et le polymère est séparé du mélange éthylène/acrylates n'ayant pas réagi par passage dans un pot de séparation.

### Conditions opératoires

➢ Débit réacteur : 4kg/h,
➢ Peroxyde utilisé: Luperox 11 (Tertio-butyl peroxypivalate) dilué dans le n-Heptane,
➢ Temps de séjour dans le réacteur: environ 50 secondes,
➢ Pression : 1900 bars (190 MPa),
➢ Monomères: mélange d'acrylates (acrylate de méthyle et glycidyle méthacrylate avec les quantités massiques respectivement de 5% et 1,5% dans le flux),
➢ Agents stabilisants: quantités variables qui sont introduites avec les monomères acryliques.

Une fois la réaction stabilisée à la température voisine de 210°C pendant environ 5 à 10min, le débit de peroxyde est ensuite progressivement augmenté toutes les 4 minutes jusqu'à ce que l'on atteigne la décomposition (parfois cette limite n'est pas atteinte), ceci permettant de déterminer les limites de décomposition (ou sensibilité à la concentration en peroxyde).

On détermine l'efficacité du stabilisant par la quantité de peroxydes injectée pour atteindre la décomposition : plus la quantité de peroxyde est élevée et plus le stabilisant est efficace.

### Essais n°1 : essais avec 230 ppm moles de stabilisant / acrylates

Dans cet essai, la vitamine E a été comparée à l'hydroxytoluène butylé (BHT) avec les mêmes concentrations molaires par rapport aux acrylates. Pour chacun des stabilisants, un minimum de 3 essais a été réalisé. La moyenne des essais a été donnée dans le tableau suivant :

| Stabilisants (composés phénoliques) | ppm mole stabilisant par rapport aux acrylates | Moyenne [C] peroxyde à la décomposition en ppm moles |
|---|---|---|
| Sans stabilisant | - | 60,4 |
| BHT | 230 | 108,8 |
| Vitamine E | 230 | 120,0 |

Les résultats montrent que la Vitamine E permet de mieux stabiliser les réactions de copolymérisation de l'éthylène car la décomposition de l'éthylène est atteinte pour des concentrations bien plus élevées en peroxydes organiques qu'avec l'hydroxytoluène butylé (BHT).

Dans ces essais, les moyennes sont significativement différentes.

### Essais n°2 : essais avec 460 ppm moles de stabilisant / acrylates

Dans cet essai, la vitamine E et le monométhyl éther d'hydroquinone (EMHQ) ont été comparés à l'hydroxytoluène butylé (BHT) avec les mêmes concentrations molaires par rapport aux acrylates. Pour chacun des stabilisants, un minimum de 3 essais a été réalisé. La moyenne des essais a été donnée dans le tableau suivant :

| Stabilisants (composés phénoliques) | ppm mole stabilisant par rapport aux acrylates | Moyenne [C] peroxyde à la décomposition en ppm moles |
|---|---|---|
| Sans stabilisant | - | 60,4 |
| BHT | 460 | 92,9 |
| EMHQ | 460 | 97,3 |
| Vitamine E | 460 | 132,5 |

Les résultats montrent que la Vitamine E et le monométhyl éther d'hydroquinone (EMHQ) permettent de mieux stabiliser les réactions de copolymérisation de l'éthylène que l'hydroxytoluène butylé (BHT) car la décomposition de l'éthylène est atteinte pour des concentrations bien plus élevées en peroxydes organiques.

Par ailleurs, les meilleurs résultats ont été obtenus avec la Vitamine E.

Dans ces essais, les moyennes sont significativement différentes.

### Essais n°3 : essais avec 115 ppm moles de stabilisant / acrylates

Dans cet essai, la vitamine E a été comparée à l'hydroxytoluène butylé (BHT) avec les mêmes concentrations molaires par rapport aux acrylates. Pour chacun des stabilisants, un minimum de 3 essais a été réalisé. La moyenne des essais a été donnée dans le tableau suivant :

| Stabilisants (composés phénoliques) | ppm mole stabilisant par rapport aux acrylates | Moyenne [C] peroxyde à la décomposition en ppm moles |
|---|---|---|
| Sans stabilisant | - | 60,4 |
| BHT | 115 | 77,2 |
| Vitamine E | 115 | 102,8 |

Les résultats montrent que la Vitamine E permet de mieux stabiliser les réactions de copolymérisation de l'éthylène que l'hydroxytoluène butylé (BHT) car la décomposition de l'éthylène est atteinte pour des concentrations bien plus élevées en peroxydes organiques.

Dans ces essais, les moyennes sont significativement différentes.

## Revendications

1. Utilisation d'au moins un composé phénolique de formule (I) suivante : 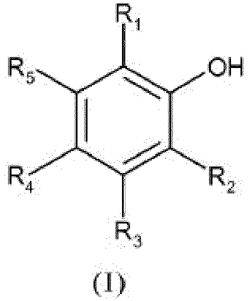 Formule (1) dans laquelle :
• R¹ représente :
- un atome d'hydrogène,
- un radical alkyle linéaire en C₁-C₈,
- un radical hydroxyalkyle linéaire en C₁-C₈,
- un groupement hydroxy,
• R², R³ et R⁵, identiques ou différents, représentent :
- un atome d'hydrogène,
- un radical alkyle en C₁-C₈, linéaire ou ramifié, éventuellement substitué par un ou plusieurs radicaux hydroxy,
- un radical alcoxy en C₁-C₈, linéaire ou ramifié, éventuellement substitué par un ou plusieurs radicaux hydroxy,
- un groupement hydroxy
• R⁴ représente :
- un radical alkyle en C₁-C₈, linéaire ou ramifié, éventuellement substitué par un ou plusieurs radicaux hydroxy,
- un radical alcoxy en C₁-C₈, linéaire ou ramifié, éventuellement substitué par un ou plusieurs radicaux hydroxy,
- un groupement hydroxy,
• étant entendu que R⁴ et R⁵ peuvent former ensemble un cycle à cinq ou six chaînons comportant un atome d'oxygène ; ledit cycle étant substitué par un radical alkyle en C₁-C₁₇, linéaire ou ramifié,
pour stabiliser les réactions de copolymérisation radicalaire de l'éthylène sous haute pression en présence d'un ou plusieurs initiateurs, le ou les initiateurs étant de préférence choisis parmi les peroxydes organiques, l'oxygène l'azobisisobutyronitrile (AIBN) et leurs mélanges, en limitant la décomposition de l'éthylène lors de réactions de copolymérisation radicalaire de l'éthylène sous haute pression.

2. Utilisation selon la revendication 1, **caractérisée en ce que**, dans la formule (1) :
• R¹ représente :
- un atome d'hydrogène,
- un radical alkyle linéaire en C₁-C₄,
• R² et R³, identiques ou différents, représentent :
- un atome d'hydrogène,
- un radical alkyle, linéaire ou ramifié, en C₁-C₅,
- un groupement hydroxy,
• R⁴ représente :
- un radical alkyle, linéaire ou ramifié, en C₁-C₅,
- un groupement hydroxy,
• R⁵ représente :
- un atome d'hydrogène,
- un radical alkyle, linéaire ou ramifié, en C₁-C₅,
- un radical alcoxy, linéaire ou ramifié, en C₁-C₅,
- un groupement hydroxy,
• étant entendu que R⁴ et R⁵ peuvent former ensemble un cycle à six chaînons comportant un atome d'oxygène ; ledit cycle étant substitué par un radical alkyle ramifié en C₁-C₁₇.

3. Utilisation selon la revendication 1 ou 2, **caractérisée en ce que**, dans la formule (1) :
• R¹, R², et R³, identiques ou différents, représentent :
- un atome d'hydrogène,
- un radical méthyle,
• R⁴ et R⁵ forment ensemble un cycle à six chaînons comportant un atome d'oxygène ; ledit cycle étant substitué par un radical alkyle ramifié en C₁-C₁₇.

4. Utilisation selon la revendication 3, **caractérisée en ce que** le ou les composés phénoliques est ou sont préférentiellement choisis parmi la formule (I') : 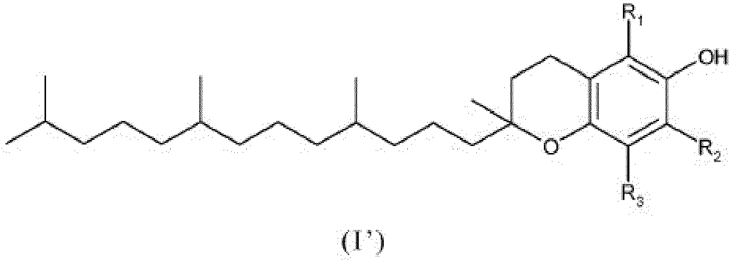 Formule (I') dans laquelle R¹, R² et R³ sont tels que définis dans la revendication 3.

5. Utilisation selon la revendication 1 ou 2, **caractérisée en ce que**, dans la formule (1) :
• R¹, R², R³ et R⁵ représentent un atome d'hydrogène et
• R⁴ représente un radical alcoxy linéaire en C₁-C₅, de préférence un radical méthoxy.

6. Utilisation selon la revendication 1 ou 2, **caractérisée en ce que**, dans la formule (1) :
• R¹ et R³ représentent un atome d'hydrogène,
• R² et R⁵ représentent un radical alkyle ramifié en C₁-C₈, de préférence en C₁-C₅ ramifié, notamment en C₄,
• R⁴ représente un groupement hydroxy.

7. Utilisation selon l'une quelconque des revendications précédentes, **caractérisée en ce que** le composé phénolique de formule (I) est choisi parmi le 2,5-di-tert-butyl hydroquinone, le 2,5-di(tert-amyl)hydroquinone, la vitamine E et le monométhyl éther d'hydroquinone, et de préférence la vitamine E et le monométhyl éther d'hydroquinone (EMHQ), encore préférentiellement est la vitamine E.

8. Utilisation selon l'une quelconque des revendications précédentes, **caractérisée en ce que** le ou les peroxydes organiques est ou sont choisis parmi les peroxy esters, les peroxydes de dialkyle, les hydroperoxydes ou les peroxycétals, en particulier les peroxydes de dialkyle, notamment le 2,5-diméthyl-2,5-di-(t-butylperoxy)hexane.

9. Utilisation selon l'une quelconque des revendications précédentes pour stabiliser les réactions de copolymérisation radicalaire avec d'autres comonomères choisis parmi les esters d'acides carboxyliques insaturés (ou leurs sels), les anhydrides d'acides carboxyliques, les esters vinyliques, les alpha-oléfines, les acides carboxyliques insaturés, les dérivés d'acides (méth)acryliques, les éthers de vinyle, les composés aromatiques vinyliques, le monoxyde de carbone et leurs mélanges.

10. Utilisation selon la revendication 9, **caractérisée en ce que** les esters d'acides carboxyliques insaturés sont choisis parmi les (méth)acrylates d'alkyles en C₁-C₂₄ et les (méth)acrylates comportant un groupement époxy.

11. Procédé de préparation de copolymères d'éthylène comprenant une étape de copolymérisation radicalaire de l'éthylène sous haute pression, de préférence dans un réacteur autoclave, en présence d'un ou plusieurs initiateurs, tels que définis selon la revendication 1 et 8, et d'un ou plusieurs composés phénoliques tels que définis selon l'une quelconque des revendications 1 à 7, le procédé étant **caractérisé en ce qu'**il ne comprend pas d'étape de préchauffage des monomères avant l'introduction dudit un ou plusieurs initiateurs.

12. Procédé de préparation de copolymères d'éthylène selon la revendication 11, **caractérisé en ce que** l'injection dudit un ou plusieurs initiateurs dans le mélange réactionnel débute à une température strictement inférieure à 100°C, de préférence à une température strictement inférieure à 80°C.

## Patentansprüche

1. Verwendung von mindestens einer phenolischen Verbindung folgender Formel (I): 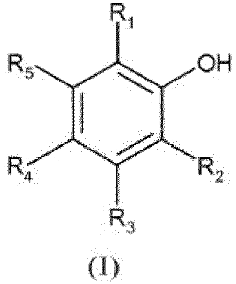 Formel (I), in der:
• R¹ Folgendes darstellt:
- ein Wasserstoffatom,
- ein lineares C₁-C₈-Alkylradikal,
- ein lineares C₁-C₈-Hydroxyalkylradikal,
- eine Hydroxygruppierung,
• R², R³ und R⁵, die gleich oder verschieden sind, Folgendes darstellen:
- ein Wasserstoffatom,
- ein lineares oder verzweigtes C₁-C₈-Alkylradikal, optional substituiert durch ein oder mehrere Hydroxyradikale,
- ein lineares oder verzweigtes C₁-C₈-Alkoxyradikal, optional substituiert durch ein oder mehrere Hydroxyradikale,
- eine Hydroxygruppierung
• R⁴ Folgendes darstellt:
- ein lineares oder verzweigtes C₁-C₈-Alkylradikal, optional substituiert durch ein oder mehrere Hydroxyradikale,
- ein lineares oder verzweigtes C₁-C₈-Alkoxyradikal, optional substituiert durch ein oder mehrere Hydroxyradikale,
- eine Hydroxygruppierung,
• mit der Maßgabe, dass R⁴ und R⁵ zusammen einen fünf- oder sechsgliedrigen Ring umfassend ein Sauerstoffatom bilden können; wobei der Ring durch ein geradkettiges oder verzweigtes C₁-C₁₇-Alkylradikal substituiert ist,
zum Stabilisieren von radikalischen Hochdruck-Copolymerisationsreaktionen von Ethylen in Anwesenheit eines oder mehrerer Initiatoren, wobei der oder die Initiatoren vorzugsweise ausgewählt sind aus organischen Peroxiden, Sauerstoff Azobisisobutyronitril (AIBN) und deren Gemischen, durch Begrenzen der Ethylenzersetzung bei radikalischen Hochdruck-Copolymerisationsreaktionen von Ethylen.

2. Verwendung nach Anspruch 1, **dadurch gekennzeichnet, dass** in der Formel (I):
• R¹ Folgendes darstellt:
- ein Wasserstoffatom,
- ein lineares C₁-C₄-Alkylradikal,
• R² und R³, die gleich oder verschieden sind, Folgendes darstellen:
- ein Wasserstoffatom,
- ein lineares oder verzweigtes C₁-C₈-Alkylradikal,
- eine Hydroxygruppierung,
• R⁴ Folgendes darstellt:
- ein lineares oder verzweigtes C₁-C₈-Alkylradikal,
- eine Hydroxygruppierung,
• R⁵ Folgendes darstellt:
- ein Wasserstoffatom,
- ein lineares oder verzweigtes C₁-C₈-Alkylradikal,
- ein lineares oder verzweigtes C₁-C₅-Alkoxyradikal,
- eine Hydroxygruppierung,
• mit der Maßgabe, dass R⁴ und R⁵ zusammen einen sechsgliedrigen Ring umfassend ein Sauerstoffatom bilden können; wobei der Ring durch ein verzweigtes C₁-C₁₇-Alkylradikal substituiert ist,

3. Verwendung nach Anspruch 1 oder 2, **dadurch gekennzeichnet, dass** in der Formel (I):
• R¹, R² und R³, die gleich oder verschieden sind, Folgendes darstellen:
- ein Wasserstoffatom,
- ein Methylradikal,
• R⁴ und R⁵ zusammen einen sechsgliedrigen Ring umfassend ein Sauerstoffatom bilden können; wobei der Ring durch ein verzweigtes C₁-C₁₇-Alkylradikal substituiert ist,

4. Verwendung nach Anspruch 3, **dadurch gekennzeichnet, dass** die phenolische(n) Verbindung(en) vorzugsweise aus der Formel (I') ausgewählt ist (sind): 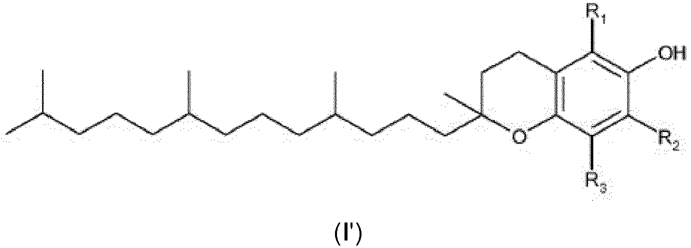 Formel (I'), wobei R¹, R² et R³ wie definiert in Anspruch 3 sind.

5. Verwendung nach Anspruch 1 oder 2, **dadurch gekennzeichnet, dass** in der Formel (I):
• R¹, R², R³ und R⁵ ein Wasserstoffatom darstellen und
• R⁴ ein lineares C₁-C₅-Alkoxyradikal, vorzugsweise einen Methoxyrest, darstellt.

6. Verwendung nach Anspruch 1 oder 2, **dadurch gekennzeichnet, dass** in der Formel (I):
• R¹ und R³ ein Wasserstoffatom darstellen,
• R² und R⁵ ein verzweigtes C₁-C₈-Alkylradikal, vorzugsweise ein verzweigtes C₁-C₅-Alkylradikal, insbesondere ein C₄-Alkylradikal, darstellen,
• R⁴ eine Hydroxygruppierung darstellt.

7. Verwendung nach einem der vorherigen Ansprüche, **dadurch gekennzeichnet, dass** die phenolische Verbindung der Formel (I) ausgewählt ist aus 2,5-di-tert-Butylhydrochinon, 2,5-di(tert-Amyl)hydrochinon, Vitamin E und Hydrochinonmonomethylether, und bevorzugt Vitamin E und Hydrochinonmonomethylether (EMHQ), bevorzugter Vitamin E ist.

8. Verwendung nach einem der vorherigen Ansprüche, **dadurch gekennzeichnet, dass** das oder die organischen Peroxide ausgewählt ist/sind aus Peroxyestern, Dialkylperoxiden, Hydroperoxiden oder Peroxyketalen, insbesondere Dialkylperoxiden, insbesondere 2,5-Dimethyl-2,5-di-(t-butylperoxy)hexan.

9. Verwendung nach einem der vorherigen Ansprüche zum Stabilisieren von radikalischen Copolymerisationsreaktionen mit anderen Comonomeren, ausgewählt aus Estern ungesättigter Carbonsäuren (oder deren Salzen), Carbonsäureanhydriden, Vinylestern, alpha-Olefinen, ungesättigten Carbonsäuren, (Meth)acrylsäurederivaten, Vinylethern, aromatischen Vinylverbindungen, Kohlenmonoxid und deren Gemischen.

10. Verwendung nach Anspruch 9, **dadurch gekennzeichnet, dass** die Ester ungesättigter Carbonsäuren ausgewählt sind aus C₁-C₂₄-Alkyl (meth)acrylaten und (Meth)acrylaten umfassend eine Epoxygruppierung.

11. Verfahren zur Herstellung von Ethylencopolymeren, umfassend einen Schritt radikalischer Copolymerisation von Ethylen unter hohem Druck, vorzugsweise in einem Autoklavenreaktor, in Anwesenheit eines oder mehrerer Initiatoren, wie definiert nach Anspruch 1 und 8, und einer oder mehrerer phenolischer Verbindungen, wie definiert nach einem der Ansprüche 1 bis 7, wobei das Verfahren **dadurch gekennzeichnet ist, dass** es keinen Schritt des Vorwärmens der Monomere vor der Einführung des einen oder der mehreren Initiatoren umfasst.

12. Verfahren zur Herstellung von Ethylencopolymeren nach Anspruch 11, **dadurch gekennzeichnet, dass** die Injektion des einen oder der mehreren Initiatoren in das Reaktionsgemisch bei einer Temperatur streng unter 100 °C, vorzugsweise bei einer Temperatur streng unter 80 °C, beginnt.

## Claims

1. - Use of at least one phenolic composition of following formula (I): 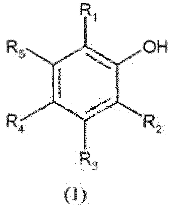 Formula (I) wherein:
• R¹ is:
- a hydrogen atom,
- a linear C₁ - C₈ alkyl radical,
- a linear C₁ - C₈ hydroxyalkyl radical,
- a hydroxy group,
• R², R³ and R⁵, the same or different, are:
- a hydrogen atom,
- a linear or branched C₁ - C₈ alkyl radical, optionally substituted by one or more hydroxy radicals,
- a linear or branched C₁ - C₈ alkoxy radical, optionally substituted by one or more hydroxy groups,
- a hydroxy group
• R⁴ is:
- a linear or branched C₁ - C₈ alkyl radical, optionally substituted by one or more hydroxy radicals,
- a linear or branched C₁ - C₈ alkoxy radical, optionally substituted by one or more hydroxy radicals,
- a hydroxy group
• on the understanding that R⁴ and R⁵ together can form a five- or six-membered ring comprising an oxygen atom; said ring being substituted by a linear or branched C₁ - C₁₇ alkyl radical,
to stabilise radical copolymerization reactions of ethylene under high pressure in the presence of one or more initiators, the initiator(s) preferably being chosen from among organic peroxides, oxygen, azobisisobutyronitrile (AIBN) and mixtures thereof, by limiting the decomposition of ethylene in radical copolymerization reactions of ethylene under high pressure.

2. - The use according to claim 1, **characterized in that** in formula (I):
• R¹ is:
- a hydrogen atom,
- a linear C₁ - C₄ alkyl radical,
• R² and R³, the same or different, are:
- a hydrogen atom,
- a linear or branched C₁ - C₅ alkyl radical,
- a hydroxy group,
• R⁴ is:
- a linear or branched C₁ - C₅ alkyl radical,
- a hydroxy group,
• R⁵ is:
- a hydrogen atom,
- a linear or branched C₁ - C₅ alkyl radical,
- a linear or branched C₁ - C₅ alkoxy radical,
- a hydroxy group,
• on the understanding that R⁴ and R⁵ together can form a six-membered ring comprising an oxygen atom; said ring being substituted by a branched C₁ - C₁₇ alkyl radical,

3. - The use according to claim 1 or 2, **characterized in that** in formula (I):
• R¹, R² and R³, the same or different are:
- a hydrogen atom,
- a methyl radical,
• R⁴ and R⁵ together form a six-membered ring comprising an oxygen atom;
said ring being substituted by a branched C₁ - C₁₇ alkyl radical,

4. - The use according to claim 3, **characterized in that** the phenolic compound(s) are preferably chosen from formula (I'): 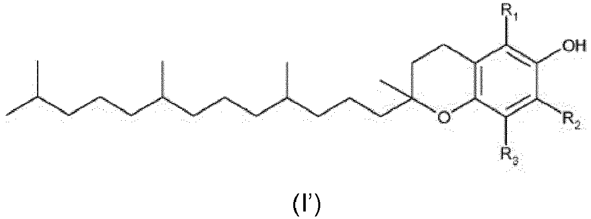 Formula (I') wherein R¹, R² and R³ are such as defined in claim 3

5. - The use according to claim 1 or 2, **characterized in that** in formula (I):
• R¹, R², R³ and R⁵ are a hydrogen atom, and
• R⁴ is a linear C₁ - C₅ alkoxy radical, preferably a methoxy radical.

6. - The use according to claim 1 or 2, **characterized in that** in formula (I):
• R¹ and R³ are a hydrogen atom,
• R² and R⁵ are a branched C₁ - C₈ alkyl radical, preferably branched C₁-C₅, in particular C₄,
• R⁴ is a hydroxy group.

7. - The use according to any of the preceding claims, **characterized in that** the phenolic compound of formula (I) is chosen from among 2,5-di-tert-butyl hydroquinone, 2,5-di(tert-amyl)hydroquinone, vitamin E and the monomethyl ether of hydroquinone, preferably vitamin E and the monomethyl ether of hydroquinone (MEHQ), and more preferably it is vitamin E.

8. - The use according to any of the preceding claims, **characterized in that** the organic peroxide(s) are chosen from among peroxy esters, dialkyl peroxides, hydroperoxides or peroxyketals, particularly dialkyl peroxides and in particular 2,5-dimethyl-2,5-di-(t-butylperoxy)hexane.

9. - The use according to any of the preceding claims, to stabilise reactions of radical copolymerization with other comonomers chosen from among the esters of unsaturated carboxylic acids (or the salts thereof), the anhydrides of carboxylic acids, vinyl esters, alpha-olefins, unsaturated carboxylic acids, derivatives of (meth)acrylic acids, vinyl ethers, aromatic vinyl compounds, carbon monoxide and mixtures thereof.

10. - The use according to claim 9, **characterized in that** the esters of unsaturated carboxylic acids are chosen from among C₁-C₂₄ alkyl (meth)acrylates and (meth)acrylates comprising an epoxy group.

11. - A method for preparing ethylene copolymers, comprising a radical copolymerization step of ethylene under high pressure, preferably in an autoclave reactor, in the presence of one or more initiators such as defined in claim 1 and 8, and one or more phenolic compounds such as defined in any of claims 1 to 7, the method being **characterized in that** it does not comprise a preheating step of the monomers before adding said one or more initiators.

12. - The method for preparing ethylene copolymers according to claim 11, **characterized in that** the injection of said one or more initiators into the reaction mixture starts at a temperature strictly lower than 100 °C, preferably at a temperature strictly lower than 80 °C.
